# EUROPEAN PATENT APPLICATION

(11) **EP 3 690 791 A1**
(43) Date of publication of application: **05.08.2020**
(21) Application number: 19783407.0
(22) Date of filing: 22.07.2019
(51) Int. Cl.: G06Q 50/00, G06Q 10/10

(54) **INFORMATION PROCESSING METHOD, PROGRAM, AND INFORMATION PROCESSING DEVICE**

(30) Priority: 18.12.2018 JP 2018236233; 17.04.2019 JP 2019078582; 17.04.2019 JP 2019078585; 17.04.2019 JP 2019078587
(71) Applicant: Finc Technologies Inc., Tokyo 1000006 (JP)
(72) Inventor: MIZOGUCHI, Yuji, 1000006 (JP); KUBOTA, Takuma Alexander, 1000006 (JP); INUKAI, Toshitaka, 1000006 (JP)
(74) Representative: Klunker IP Patentanwälte PartG mbB
(86) International application number: PCT/JP2019/028620
(87) International publication number: WO 2020/129292

(57) **Abstract**

The present disclosure relates to communications between users in a community service within a virtual space, and provides an information processing method, a program, and an information processing device which facilitate activating communities through the mediation of a virtual user. In this method, users including an administrator user and a plurality of general users are associated with a virtual user for supporting the users in a community. The method includes the steps, executed by a computer, of generating an event associated with the administrator user and a first general user; and in response to the generated event, causing the virtual user to generate prompt content for prompting a second general user to communicate with the first general user, and supply the prompt content to the second general user in the community.

## Description

### TECHNICAL FIELD

The present disclosure relates to an information processing method, a program, and an information processing device. More specifically, the present disclosure relates to an information processing method, a program, and an information processing device for activating communities in community services.

### BACKGROUND ART

A variety of community services are known, including SNS (Social Network service) on the Internet. One of known prior-art community services is an electric bulletin board or chat service (Patent Document 1). Recently, there are also known community services such as fan club community services established by celebrities, content live delivery service, and on-line salon service.

A plurality of members (users) belong to a community service. Users are categorized into an operator who provides the community service (administrator user that may refer to, for example, creators, personalities, athletes, celebrities, and the like), and customers (general users) that utilize the community service. In other words, a community service is organized by one administrator user (or one administrator user account) and N general users (or N customer accounts, where N is generally an integer equal to or larger than two) who are served by the one administrator user.

In a community service, an administrator user generally provides a variety of content. General users, in turn, make responses (reactions) to the provided content, and the administrator user and other general users make reactions to the action of the general user, thus resulting in communications between the administrator user and general users and between the general users. The community service is such that the general users pay service fees (for example, monthly membership fees) and content usage charge to the administrator user who serves to maintain the operation of the community service.

In relation to the community service, an instant messaging (IM) service is known as one implementation thereof. Some IM services allow a plurality of users to organize a group, using terminals such as smart phones and tablets, for implementing messaging within the group, thereby enabling dialogues to be made among one another.

Specifically, a user may install an application for IM service into his portable terminal, and link accounts with each other (become "friends"), allowing for the user to perform messaging through a talk room which provides a dedicated virtual space community. Here, a founder of a group serves as an administrator user, while users joined into the group by the founder are designated general users.

Also, some of such IM services allow interactive automated dialogues between a user and a virtual user also referred to as "bot" (Patent Document 2). The prior-art bot user acts to support a particular user by making a one-to-one communication between the bot user and the particular user.

### CITATION LIST

### PATENT DOCUMENT

[Patent Document 1] JP-A-2001-144754
[Patent Document 2] JP-A-2018-101352

### SUMMARY OF INVENTION

### TECHNICAL PROBLEM

Currently, community services encounter a variety of issues. For example, increasing the number of general users is desirable for an administrator user as a business, whereas a larger number of customers requires more efforts and cost for the operation of the community service. Also, in such a situation, the administrator user is loaded with great efforts in making further reactions to reactions of general users. Further, general users may often hesitate reactions since they assume that their own reactions can be a trigger to destroy the community. The general users also often hesitate to disclose their personal information to the community.

As a result of a community organized by one administrator user and N general users served by the one administrator user, although the community itself is open, actually, a majority of services often stay in unilateral information delivery from the administrator user. In other instances, the automated dialogue with a bot user in the IM service, though an artificial intelligence is implemented, merely provides limited functionality in a current status. More specifically, a communication of one user is simply supported in a limited manner using a particular function (for example, an automated response function or the like).

It is an object of the present disclosure to solve at least part of the above-mentioned issues. Specifically, the present disclosure relates to communications between users in a community service within a virtual space, and one object thereof is to activate a community through the mediation of virtual users. Particularly, one object of the disclosure is to support maintenance and improvement of relationships between an administrator user and general users and between general users. Another object of the present disclosure is to provide an administrator user with an effective operation of community services within a virtual space through the mediation of the virtual users. A further object of the present disclosure is to encourage general users to utilize community services without hesitation through the mediation of the virtual users.

### SOLUTION TO PROBLEM

According to one embodiment of the disclosure, an information processing method is provided, wherein a community is associated with users including an administrator user and a plurality of general users, and a virtual user for supporting the users. The method includes the steps, executed by a computer, of generating an event associated with the administrator user and a first general user; and in response to the generated event, causing the virtual user to generate prompt content for prompting a second general user to communicate with the first general user, and supply the prompt content to the second general user in the community.

Also, according to another embodiment of the disclosure, a program is provided for causing a computer to execute the information processing method.

Further, according to a further embodiment of the disclosure, an information processing device is provided. The information processing device includes a management unit for associating users including an administrator user and a plurality of general users with a virtual user for supporting the users in a community; an event generation unit for generating an event associated with the administrator user and a first general user; and a prompt content supply unit for causing the virtual user to, in response to the generated event, generate prompt content for prompting a second user to communicate with the first general user, and supplying the prompt content to the second general user in the community.

With each of the embodiments of the disclosure, communities can be activated through autonomous mediation of a virtual user in a community service within a virtual space. Particularly, by supporting maintenance and improvement of the relationship between an administrator user and general users, and between general users, the number of members belonging to a community can be effectively increased, accompanied with improvement in active user ratio. Also, by providing the administrator user with an effective operation of community services through the mediation of the virtual user, the administrator user can be relieved from burdens involved in the maintenance and management of the community. Further, general users can be encouraged to utilize community services without hesitation, through the mediation of the virtual user, obviating such situations as a troubled community, and concentrated attacking to a particular general user. In addition, unnecessary private data of general users can be prevented from being disclosed.

### BRIEF DESCRIPTION OF DRAWINGS

Fig. 1 is a basic configuration diagram of an information processing system.
Fig. 2 is a hardware configuration diagram of an information processing device equipped in the information processing system.
Fig. 3 is a hardware configuration diagram of a user terminal equipped in the information processing system.
Fig. 4 is a functional block diagram of an exemplary information processing system which is implemented in accordance with one embodiment.
Fig. 5 is a conceptual diagram illustrating a relationship between users in a community.
Fig. 6 is a conceptual diagram illustrating a relationship between a virtual user and a user, and a concept of a talk room.
Fig. 7 is an exemplary flow diagram showing a processing flow executed by one embodiment.
Fig. 8 is an exemplary flow diagram showing a processing flow executed by one embodiment.
Fig. 9 is an exemplary flow diagram showing a processing flow executed by one embodiment.
Fig. 10 is a schematic diagram showing an exemplary screen image generated by one embodiment.
Fig. 11 is a schematic diagram showing an exemplary screen image generated by one embodiment.
Fig. 12 is a schematic diagram showing an exemplary screen image generated by one embodiment.

### DESCRIPTION OF EMBODIMENTS

First, an illustrative embodiment of the disclosure will be described while listing components thereof. An information processing method, a program, and an information processing device, according to an embodiment of the disclosure, may comprise the following configuration.
(Aspect 1) According to Aspect 1, an information processing method is provided, wherein a community is associated with users including an administrator user and a plurality of general users, and with a virtual user for supporting the users, the method includes the steps, executed by a computer, of generating an event associated with the administrator user and a first general user; and in response to the generated event, causing the virtual user to generate a prompt content for prompting a second general user to communicate with the first general user, and supply the prompt content to the second general user in the community.
(Aspect 2) According to Aspect 2, the method of Aspect 1 further includes the step of supplying reaction content created by the second general user in response to the prompt content supplied thereto to the first general user in the community.
(Aspect 3) According to Aspect 3, the method of Aspect 1 or 2, further includes the steps of accepting a registration of a mission by the administrator user, associated with the community; accepting an action taken on the mission by the first general user; and determining the achievement of the mission in accordance with the reception of the action, wherein an event is generated in accordance with the achievement of the mission.
(Aspect 4) According to Aspect 4, the method of Aspects 1 - 3, further includes the steps of supplying question content created by the first general user to the administrator user in the community; and supplying answer content created by the administrator user in response to the question content supplied to the first general user in the community, wherein an event is generated in accordance with the establishment of a question-and-answer session made up of the question content and answer content.
(Aspect 5) According to Aspect 5, in the method of Aspects 1 - 4, a virtual-space community is implemented as a talk room by an instant messaging application, wherein messaging by the second general user is displayed on a first column, and messaging by the administrator user and virtual user is displayed on a second column in a time-series order on a screen of the talk room displayed on the terminal of the second general user.
(Aspect 6) According to Embodiment 6, in the method of Aspect 5, the messaging by the first general user is further displayed on the second column in association with the messaging by the virtual user or administrator user on the screen of the talk room displayed on the terminal of the second general user.
(Aspect 7) According to Aspect 7, a program is provided for causing a computer to execute the methods of Aspects 1 through 8.
(Aspect 8) According to Aspect 8, an information processing device is provided. The information processing device comprises a management unit for associating users including an administrator user and a plurality of general users with a virtual user for supporting the users in a community; an event generation unit for generating an event associated with the administrator user and a first general user; and a prompt content supply unit for causing the virtual user to generate prompt content for prompting the second general user to communicate with the first general user in response to the generated event, and to supply the prompt content to the second general user in the community.
(Aspect 9) According to Aspect 9, the information processing device of Aspect 8, further comprises a reaction content supply unit for supplying reaction content created by the second general user in response to the prompt content supplied to the first general user in the community.
(Aspect 10) According to Aspect 10, the information processing device of Aspect 8 or 9, further comprises a mission registration unit for accepting a registration of a mission by the administrator user associated with the community; a reception unit for receiving an action taken on the mission by the first general user; and a determination unit for determining the achievement of the mission in response to the received action, wherein an event is generated in accordance with the achievement of the mission in the event generation unit.
(Aspect 11) According to Aspect 11, the information processing device of Aspects 8 through 10, further includes question content supply unit for supplying question content created by the first general user to the administrator user in the community; and an answer content supply unit for supplying answer content created by the administrator user in response to the question content supplied to the first general user in the community, wherein an event is generated in the event generation unit in accordance with the establishment of a question-and-answer session achieved by the question content supply unit and the answer content supply unit.
(Aspect 12) According to Aspect 12, an information processing method is provided, wherein a community is associated with users including an administrator user and a plurality of general users, and with a virtual user for supporting the users, the method includes the steps, executed by a computer, of generating an event associated with the administrator user and a first general user, in accordance with an action taken by the first general user; and, in response to the generated event, causing the virtual user to generate prompt content for prompting the second general user to communicate with the first general user, and supply the prompt content to the second general user in the community.
(Aspect 13) According to Aspect 13, a method of Aspect 12, further includes the step of supplying reaction content created by the second general user in response to the prompt content supplied to the first general user in the community.
(Aspect 14) According to Aspect 14, a method of Aspect 12 or 13, further includes the steps of receiving a registration of a mission associated with the community by the administrator user; accepting an action taken on the mission by the first general user; and determining the achievement of the mission in response to the received action, wherein an event is generated in accordance with the achievement of the mission.
(Aspect 15) According to Aspect 15, in the method of any of Aspects 12 through 14, the community is implemented as a talk room by an instant messaging application, wherein messaging by the second general user is displayed on a first column, and messaging by the administrator user and virtual user is displayed on a second column in a time-series order on a screen of the talk room displayed on the terminal of the second general user.
(Aspect 16) According to Aspect 16, in the method of Aspect 15, messaging by the first general user is displayed on the screen of the talk room displayed on a terminal of the second general user on the second column in association with the messaging by the virtual user or the administrator user.
(Aspect 17) According to Aspect 17, an information processing method is provided, wherein a community is associated with users including an administrator user and a plurality of general users, and with a virtual user for supporting the users, the method including the steps, executed by a computer, of generating an event associated with the administrator user and the first general user, in accordance with an action taken by a first general user, wherein history information on the taken action is registered by a terminal of the first general user; and in response to the generated event, causing the virtual user to generate a prompt content for prompting a second general user to communicate with the first general user, and supply the prompt content to the second general user in the community.
(Aspect 18) According to Aspect 18, the method of Aspect 17, further includes the step of supplying a reaction content created by the second general user in response to the prompt content supplied to the first general user in the community.
(Aspect 19) According to Aspect 19, a method of Aspect 17 or 18, further includes the steps of accepting a registration of a mission associated with the community by the administrator user; accepting an action taken on the mission by the first general user; and determining the achievement of the mission in response to the received action, wherein an event is generated in accordance with the achievement of the mission.
(Aspect 20) According to Aspect 20, in the method of any of Aspects 17 through 19, the community is implemented as a talk room by an instant messaging application, wherein messaging by the second general user is displayed on a first column, and messaging by the administrator user and virtual user is displayed on a second column in a time-series order on a screen of the talk room displayed on a terminal of the second general user.
(Aspect 21) According to Aspect 21, in the method of Aspect 20, the messaging by the first general user is further displayed on the second column in association with the messaging by the virtual user or the administrator user on the screen of the talk room displayed on the terminal of the second general user.
(Aspect 22) According to Aspect 22, an information processing method is provided, wherein a community is associated with users including an administrator user and a plurality of general users, and with a virtual user for supporting the users, the method including the steps, executed by a computer, of generating an event associated with the administrator user and a first general user, in accordance with an action taken by the administrator user; and in response to the generated event, causing the virtual user to generate a prompt content for prompting a second general user to communicate with the first general user, and supply the prompt content to the second general user in the community.
(Aspect 23) According to Aspect 23, the method of Aspect 22, further includes the step of supplying a reaction content created by the second general user in response to the prompt content supplied to the first general user in the community.
(Aspect 24) According to Aspect 24, the method of Aspect 22 or 23, further includes the steps of supplying question content created by the first general user to the administrator user in the community; and supplying answer content created by the administrator user in response to the question content supplied to the first general user in the community, wherein an event is generated in accordance with the establishment of a question-and-answer session made up of the question content and the answer content.
(Aspect 25) According to Aspect 25, in the method of any of Aspects 22 through 24, the community is implemented as a talk room by an instant messaging application, wherein messaging by the second general user is displayed on a first column, and messaging by the administrator user and virtual user is displayed on a second column in a time-series order on a screen of the talk room displayed on a terminal of the second general user.
(Aspect 26) According to Aspect 26, in the method of Aspect 25, the messaging by the first general user is further displayed on the second column in association with the messaging by the virtual user or administrator user on the screen of the talk room displayed in the terminal of the second general user.

In the following, embodiments of an information processing method, a program, and an information processing device according to the present disclosure will be described in conjunction with the accompanying drawings, where the same or similar elements are designated the same or similar reference symbols. In the description of embodiments, repetitive descriptions on the same or similar elements may be omitted. Also, features shown in embodiments can be applied to other embodiments unless they are inconsistent to each other.

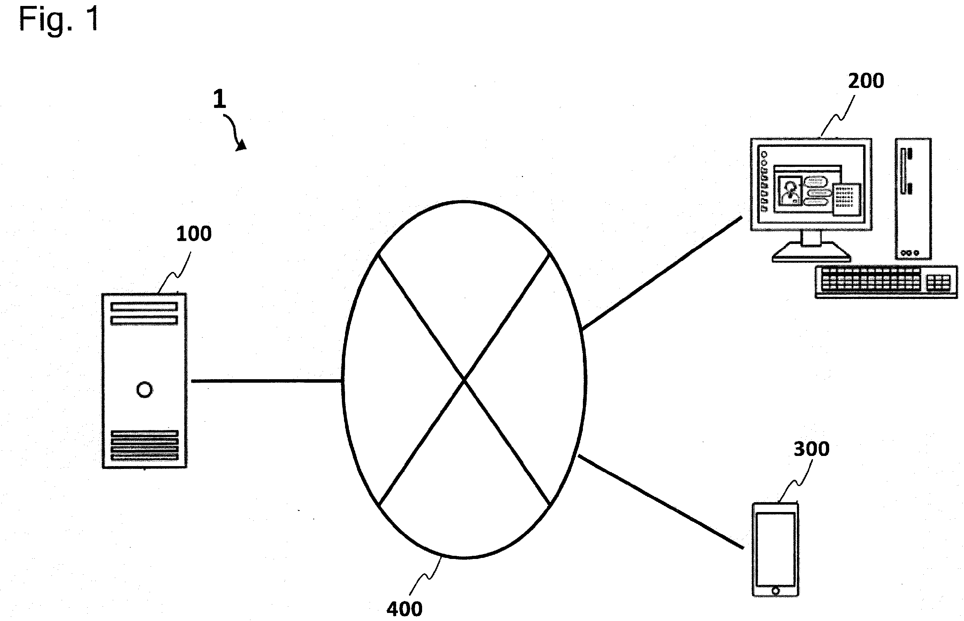

Fig. 1 is a general conceptual diagram of an information processing system 1 which comprises a server 100, and a plurality of user terminals 200, 300. The server 100 is connected to the user terminals 200, 300 over a network 400. While only two user terminals are shown in Fig. 1 for facilitating the description, this is not a limitation, and three or more user terminals may be provided. Also, the user terminals 200, 300 are not limited to a personal computer and a smart phone as illustrated, but, other than those, may be an electronic device, such as, for example, a laptop computer, a future phone, PDA (Personal Digital Assisant), and a tablet terminal.

In the following description of embodiments, a community service is assumed to be provided by an instant messaging (IM) application. Specifically, in a talk room implemented by an IM application installed in the user terminals 200, 300, respectively, communications are made by transmitting/receiving a variety of content between an administrator user and a general user and between general users.

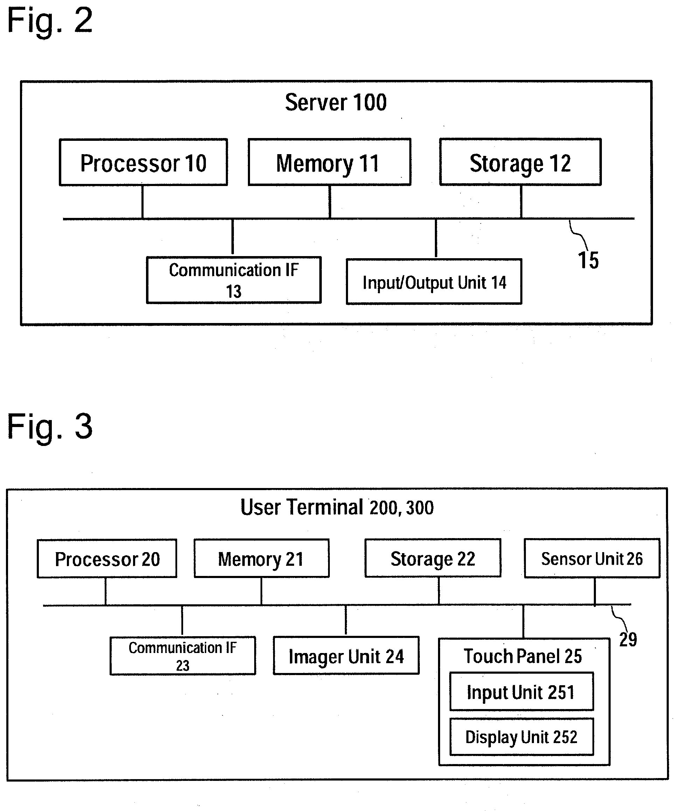

Referring to Figs. 2 and 3, the server 100 and user terminals 200, 300 will be described in regard to the hardware configuration. Fig. 2 is a hardware configuration diagram of the server 100. The server 100 is an information processing device according to one embodiment for providing services through the IM application, and may be implemented by a general-purpose computer, such as a workstation and a personal computer, by way of example. Alternatively, the server 100 may be logically implemented by cloud computing. The server 100 comprises a processor 10, a memory 11, a storage 12, a communication interface (IF) 13, an input/output unit 14, and the like, all of which are electrically interconnected through a bus 15.

The processor 10 is a processing unit for generally controlling the operation of the server 100, and performing information processing and the like required for control of data transmission/reception between respective components and for execution of applications. For example, the processor 10 is a CPU (Central Processing Unit) which executes programs stored in the storage 12 and extended into the memory 11 to perform appropriate information processing.

The memory 11 includes a main storage comprised of a volatile storage device such as DRAM (Dynamic Random Access Memory), and an auxiliary storage comprised of a non-volatile storage device such as a Flash memory and HDD (Hard Disc Drive). The memory 11 is used as a work area and the like for the processor 10, and stores BIOS (Basic Input/Output System) executed upon startup of the server 100, a variety of setting information, and the like.

The storage 12 stores a variety of programs such as application programs and a user authentication program. A database may be built in the storage 12 for storing data used in appropriate processing of the IM application.

The communication interface 13 connects the server 100 to the network 400 to make communications with the user terminals 200, 300. The input/output unit 14 may be an information input device such as a mouse and a keyboard, and an output device such as a display. The bus 15 is commonly connected to the respective components described above, to communicate, for example, an address signal, a data signal, and a variety of control signals.

Fig. 3 is a hardware configuration diagram of the user terminals 200, 300. The example illustrated in Fig. 3 is assumed to be a smart phone (300) having a touch panel, as illustrated in Fig. 1, though it is not so limited. The user terminal 200, 300 comprises a processor 200, a memory 21, a storage 22, a communication interface 23, an imager unit 24, a touch panel 25, a sensor unit 26, and the like, all of which are electrically interconnected through a bus 29.

The processor 20 is a processing unit for controlling the operation of the user terminal 200, 300, and performing processing and the like required for control of data transmission/reception between the respective components, and for execution of applications. For example, the processor 20 is a CPU and/or GPU (Graphical Processing Unit) or the like, and executes required information processing by executing programs and the like stored in the storage 22 and extended into the memory 21.

Memory 21 includes a main storage comprised of a volatile storage device such as RAM, and an auxiliary storage comprised of a non-volatile storage device such as a Flash memory and HDD. The memory 21 is used as a work area and the like for the processor 20, and stores BIOS executed upon startup of the sever 100, a variety of setting information, and the like. The storage 22 stores application programs and the like.

The communication interface 23 connects the user terminal 200, 300 to the network 400 to make communications with the server 100. The communication interface 13 also includes a short-range communication interface such as Bluetooth (Registered Trademark) and BLE (Bluetooth Low Energy). The imager unit 24 has a camera function, and stores images taken by the user in the storage 22. The touch panel 25 comprises an input unit 251 and a display unit 252.

The input unit 251 of the touch panel senses a manipulation made by the user on the touch panel 25. The input unit 251 may employ a pressure sensing scheme, a resist film scheme, a capacitive scheme, an electromagnetic induction scheme, or the like. Then, the input unit 251 receives an input through a manipulation at an arbitrary position on the touch panel (a physically touching manipulation on the touch panel such as touching, sliding, swiping, and tapping). In this event, a changing amount of pressing, electric resistance, electric capacitance, energy of elastic wave, and the like is sensed at a touched position, to identify the corresponding coordinates of the touched position. The display unit 252 of the touch panel is made of a liquid crystal display or the like.

The sensor unit 26 may include, but not limited to, an acceleration sensor, a vibration sensor, a gyro-sensor, a geomagnetic sensor, a proximity sensor, an illuminance sensor, a pressure sensor, a GPS, and the like, as a built-in sensor. As an example, the sensor unit 26 functions as an activity monitor for measuring activity information including the number of steps, a travelling distance, an activity range, and the like of the user. Additionally, the activity information may be received through the communication interface 23 in association with an external wearable device (not shown). By using an external wearable device, more detailed bio-information can be captured through an ECG (electrocardiogram) sensor and a myoelectric sensor. Other than the above, a variety of data may be captured from a meteorological sensor or the like. The bus 29 is commonly connected to the respective components listed above, to communicate, for example, an address signal, a data signal, and a variety of control signals.

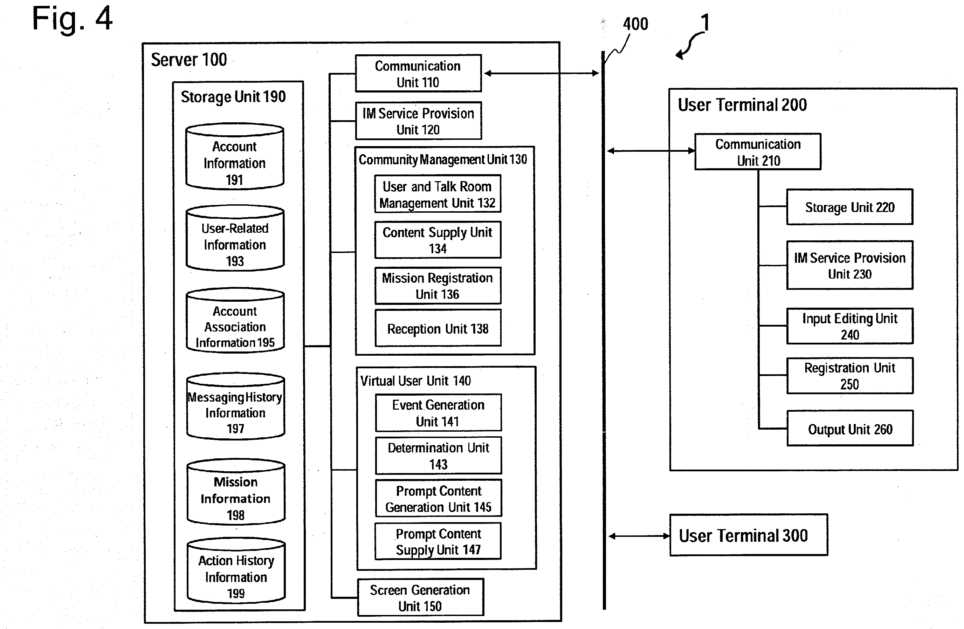

Referring to Fig. 4, the information processing system 1 will be described in regard to its functions and configuration. The information processing system 1 comprises an information processing device according to one embodiment and configured for implementing a community as a talk room through an instant messaging (IM) application. Fig. 4 is an exemplary block diagram showing functions implemented in the server 100 and user terminals 200, 300, respectively.

The server 100 comprises a communication unit 110, an IM service provision unit 120, a community management unit 130, a virtual user unit 140, a screen generation unit 150, and a storage unit 190, all of which interact with one another. The user terminal 200, 300 comprises a communication unit 210, a storage unit 220, an IM service provision unit 230, an input editing unit 240, a registration unit 250, and an output unit 260, all of which interact with one another. The user terminal 300 is similar to the user terminal 200 in function and configuration (hereinafter, the user terminals 200, 300 are collectively referred to as the "user terminal 200" unless particularly noted). In this regard, it is assumed herein that the user terminal 200 is a terminal of a general user, while the user terminal 300 is a terminal for an administrator user.

The communication unit 110 of the server 100 connects the server 100 to the network 400 to transmit/receive data through communications with the user terminal 200. The IM service provision unit 120 provides basic functions for implementing an IM application to realize messaging by a plurality of users. The community management unit 130 provides a function for managing a community as a talk room in the IM service. The virtual user unit 140 implements a virtual user function for assisting the user to activate the community. The screen generation unit 150 generates a user screen associated with the talk room. The respective components will be described below in greater detail.

The IM service provision unit 120 provides an IM application function. Information on a user who utilizes the IM application may be included in account information 191 and user-related information 193. The user generically includes an administrator user and a plurality of general users who belong to a community. The users can be directly linked to each other as "friends," or can be indirectly linked through a community by belonging to the same community. Information on the link may be included in account association information 195.

The community management unit 130 further comprises a user/talk room management unit 132, a content supply unit 134, a mission registration unit 136, and a reception unit 138. The user/talk room management unit 132 of the community management unit 130 associates a user with a virtual user that supports the user in the talk room. The associated information may be included in the account association information 195. The user/talk room management unit 132 also manages the history of messaging in the talk room. The managed history information may be included in messaging history information 197.

The content supply unit 134 of the community management unit 130 performs processing related to messaging into the talk room by the user. The content supply unit 134 further comprises question content supply unit, an answer content supply unit, and a reaction content supply unit (all of which are not shown). The question content supply unit supplies question content generated by a general user to the talk room of the administrator user in the community. The answer content supply unit supplies answer content generated by the management user in response to the question content supplied to the talk room of the general user who is the questioner, in the community. The reaction content supply unit supplies reaction content generated by a general user in response to prompt content supplied from the virtual user to the talk room of another general user in the community. Information related to the supplied content may be included in the messaging history information 197.

The mission registration unit 136 of the community management unit 130 accepts a registration of a mission from the registration unit 250 of the terminal 300 of the administrator user. Information on the mission may be included in mission information 198 so as to be associated with the administrator user.

The reception unit 138 of the community management unit 130 receives an action of a general user on a mission registered by the administrator user in the mission registration unit 134. Information on the action may be included in action history information 199 so as to be associated with the general user.

The virtual user unit 140 further includes an event generation unit 141, a determination unit 143, a prompt content generation unit 145, and a prompt content supply unit 147. The event generation unit 141 of the virtual user unit 140 causes a virtual user to generate an event associated with the administrator user and a general user in the community. In one example, the event may be generated in accordance with the achievement of a mission, as determined in the determination unit 143. Alternatively, the event may be generated in response to answer content supplied from the administrator user by the content supply unit 134 (answer content supply unit).

The determination unit 143 of the virtual user unit 140 causes the virtual user to determine the achievement of a mission registered by the administrator user in the mission registration unit 136, with reference to information on an action on the mission, taken by a general user and received at the reception unit 138.

The prompt content generation unit 145 of the virtual user unit 140 performs processing for generating content commensurate with the generation of an event in the event generation unit 141. The generated content is, for example, prompt content for the virtual user to prompt a distinct general user to make a communication between a general user who has achieved a mission (associated with the event) and this distinct general user.

The prompt content supply unit 147 of the virtual user unit 140 causes the virtual user to supply prompt content generated in the prompt content generation unit 145 to a talk room of another general user in the community. The supply of the prompt content to the talk room may be controlled in timing in accordance with how the community is congested. For example, assume that if prompt content were supplied to the talk rooms of many general users at one time, reaction content would be concentrated for the prompt content. In this case, the supply of the prompt content may be controlled such that the timing is shifted in accordance with the intimacy between general users. Alternatively, the prompt content may be controlled such as to be supplied only to those users having a high intimacy.

The virtual user unit 140 may comprise, for example, a content censoring unit (not shown), in addition to the foregoing components. The content censoring unit censors whether content (text information, image information, links to web pages, and the like) supplied from the administrator user and general users contains inadequate contents in the community.

The screen generation unit 150 generates a user screen of the talk room using a variety of information stored in the storage unit 190. The user screen is generated for each of the user terminals 200 of the respective general users and the user terminal 300 of the administrator user. Thus, the generated screens are different on the respective displays of the users. Generally, content is displayed in a time-series order such that content supplied by a user himself to the talk room is arranged along the right side of the screen, while content received from other users than himself and virtual user is arranged along the left side of the screen, though this is not limitations. The user can review past content in the talk room backward through the transmission/reception history by scrolling the screen.

The storage unit 190 manages the account information 191, user-related information 193, account association information 195, messaging history information 197, mission information 198, and action history information 199, as described above. In the storage unit 190, a variety of information may be managed as records, for example, in a tabular-formatted database. As the community is activated with active messaging originated from users, a variety of more information will be registered and/or updated. In addition to the foregoing information, the storage unit 190 may include a variety of data files, and programs which implement IM applications and virtual user functions. Stated another way, the storage unit 190 can store a program which implements an information processing method according to one embodiment.

The communication unit 210 of the user terminal 200 connects the user terminal 200 to the network 400 to transmit/receive data through communications with the server 100. The storage unit 220 stores a variety of data and programs related to IM applications required to utilize IM services on the user terminal. The IM service provision unit 230 executes the IM application to enable messaging by users in the talk room. The input editing unit 240 enables messages to be input and/or edited through the input unit 251 upon messaging.

The registration unit 250 registers a mission registered in the mission registration unit 136 of the server 100, or an action received at the reception unit 138 of the server 100. The registration of an action in the registration unit 250 may include a data registration through a cooperation with an external device such as a wearable device which cooperates with the user terminal 200. The output unit 260 outputs a user screen generated by the screen generation unit 150 of the server 100 to the display unit 252.

The respective components in the respective hardware configurations of the foregoing server 100 and user terminals 200, 300 illustrated in Figs. 2 and 3, as well as the respective function blocks illustrated in Fig. 4 are merely illustrative, and the present disclosure is not limited to those. Also, each function block illustrated in Fig. 4 may be implemented as a portion of hardware, and/or a portion of software. Further, at least part of the respective function blocks 110 - 190 of the server 100 illustrated in Fig. 4 may be included in the user terminal 200. On the contrary, at least part of the respective functions 210 - 260 of the user terminals 200, 300 may be configured to be included in the server 100. In other words, the information processing method and program according to one embodiment can be executed by any device selected from the server 100 and user terminals 200, 300.

The following description will be given on the outline of an exemplary community service implemented by the information processing method, program, and information processing device according to one embodiment. Here, the community service is assumed to be a health-care service through an instant messaging (IM) application. Particularly, a community named "Diet Group" exists, where each user is provided with a talk room by the IM application. An image of the talk room is displayed on the user terminal 200.

In the "Diet Group" community, messaging is progressed between at least one instructor, who is a nutritionist, and two or more healthcare seeking persons, and/or between healthcare seeking persons through talk rooms. Assume herein that the instructor serves as an administrator user who administrates the community, while a plurality of healthcare seeking persons are general users who utilize the community service.

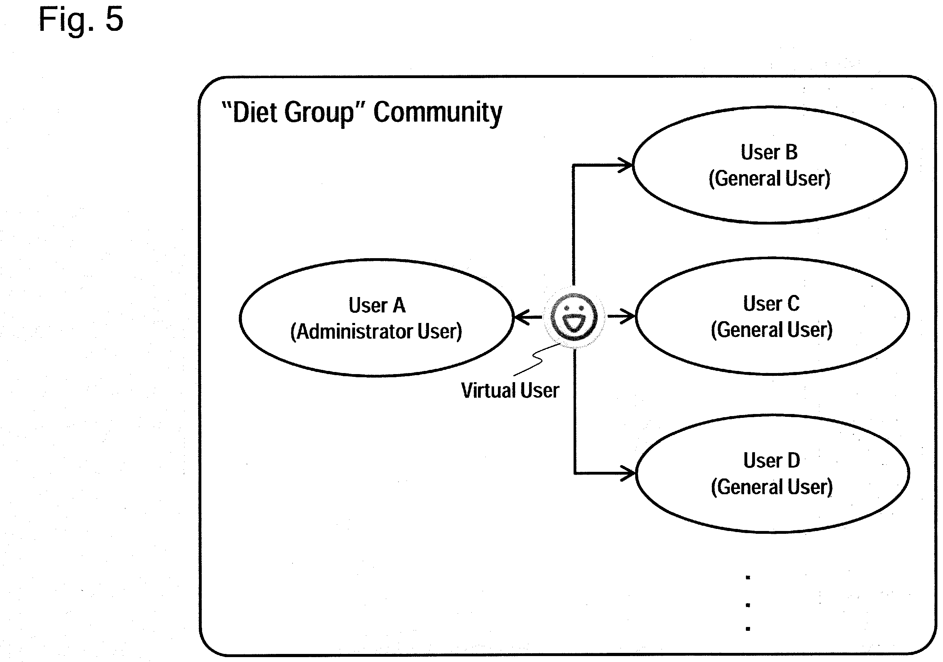

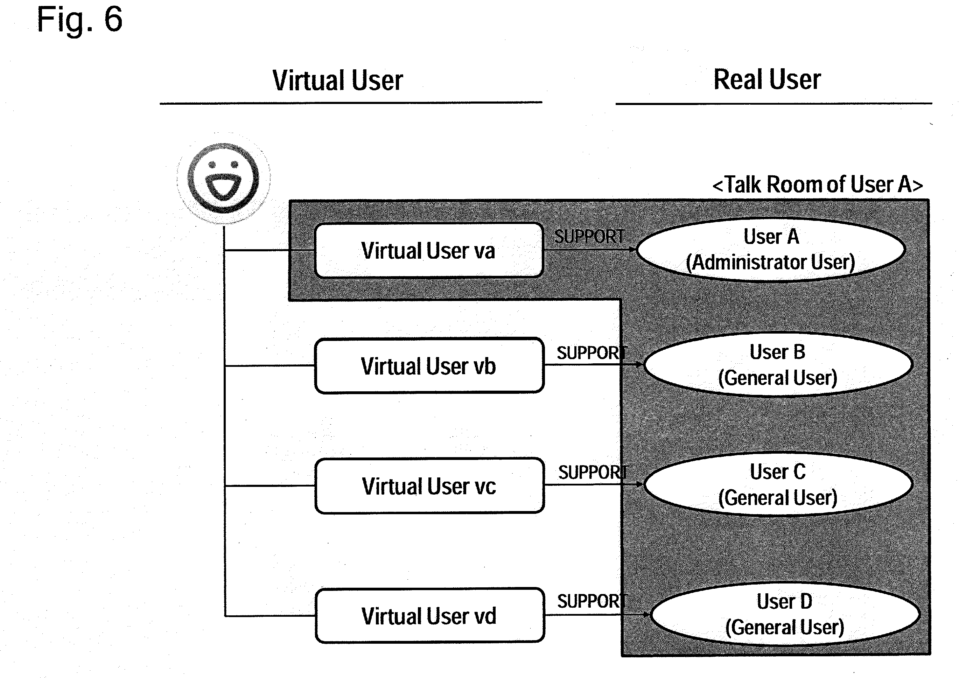

Fig. 5 is an exemplary conceptual diagram related to an association of accounts of a plurality of users who have downloaded the IM application, registered their accounts, or participate in the "Diet Group" community. Fig. 6 in turn is an exemplary conceptual diagram related to the relationship between real users participating in the "Diet Group" community and a virtual user, and related to the talk room which is generated based on the relationship. The association in Fig. 5 and the relationship and talk room in Fig. 6 are managed by the users and talk room management unit 132.

As shown in Fig. 5, User A - User D belong to the "Diet Group" community. User A is an instructor who may be an administrator user, while User B - User D are healthcare seeking persons who are general users. Mediating between the respective users is a virtual user which is implemented by the virtual user unit 140 and displayed in the form of icon. The virtual user autonomously operates to support communications of the respective users by mediating the communications between Users A - D. Specifically, through autonomous mediating operations of the virtual users, active communications are prompted between the administrator user A and general users B - D and further, active communications are also prompted between the general users B - D. In this way, effective activation of the community can be achieved. It should be understood that the "Diet Group" community may run with users belonging thereto, including at least one administrator user and a plurality of general users, and is not limited to four users A - D.

As shown in Fig. 6, the virtual users implemented by the virtual user unit 140 and displayed in the form of icon comprise virtual users va - vd for each of the users A - D. Thus, the virtual users va - vd support the users A - D, respectively. As a result, in the talk room of the user A, for example, messaging by the users A - D and virtual user va is managed as illustrated. Similarly, messaging by the users A - D and virtual user vb is managed in the talk room of the user B; messaging by the users A - D and virtual user vc is managed in the talk room of the user C; and messaging by the user A - D and virtual user vd is managed in the talk room of the user D.

As described above, in one embodiment, the community service is assumed to be a healthcare service which is implemented as a talk room by an instant messaging (IM) application. The messaging refers to transmission/reception of content including a message and a stamp image. The content can also include, in addition to text information and image information, part or all of a web page, an audio file, a video file, and the like. A talk room, the community name of which is designated as the "Diet Group," is displayed on each of the terminals possessed by Users A - D. In this regard, the talk room may also comprise sub-communities derived from the "Diet Group" community, such as "Summer Slimming Diet" and "Winter Fat Cancellation", by way of example.

It should be understood that the embodiment is not limited to the details of the community or the type of content. Also, not limited to the IM application, any application can be employed as long as it is capable of linking accounts of a plurality of users to one another to transmit/receive content among the accounts, such as SNS and blog. In other words, the embodiment can be widely applied to communications among users in communities within virtual spaces on the Internet.

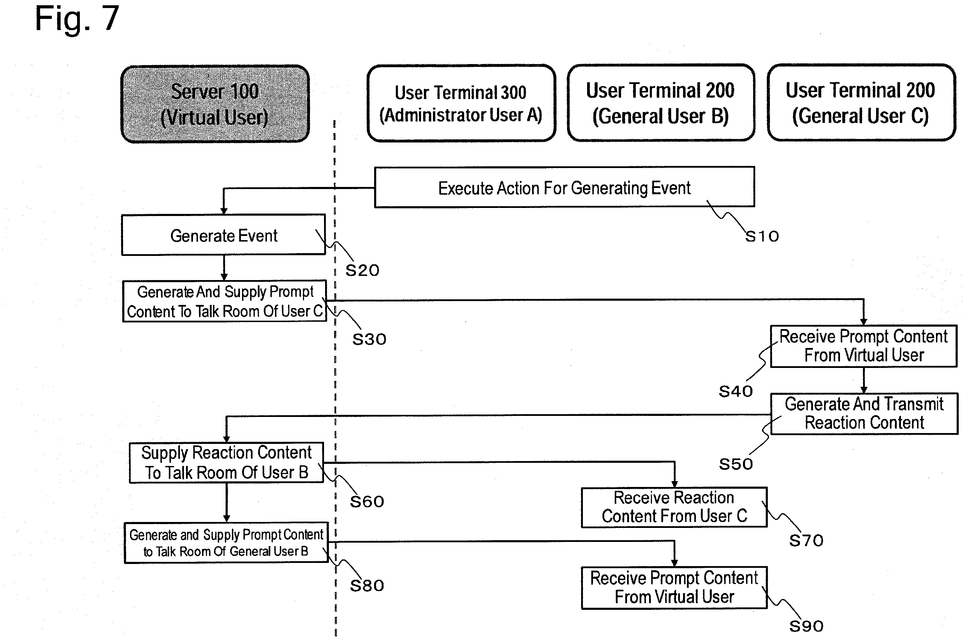

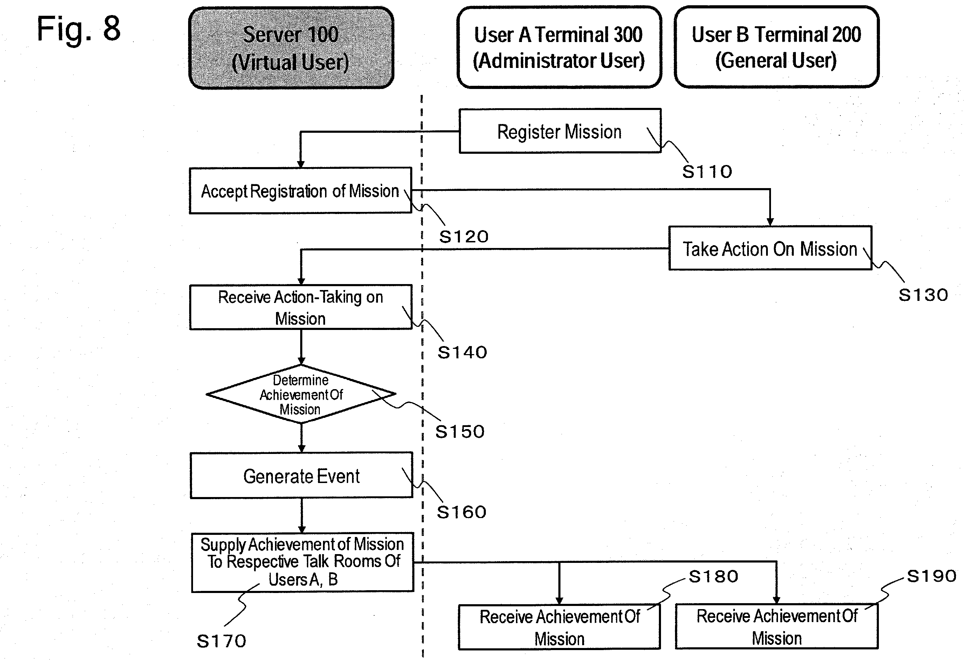

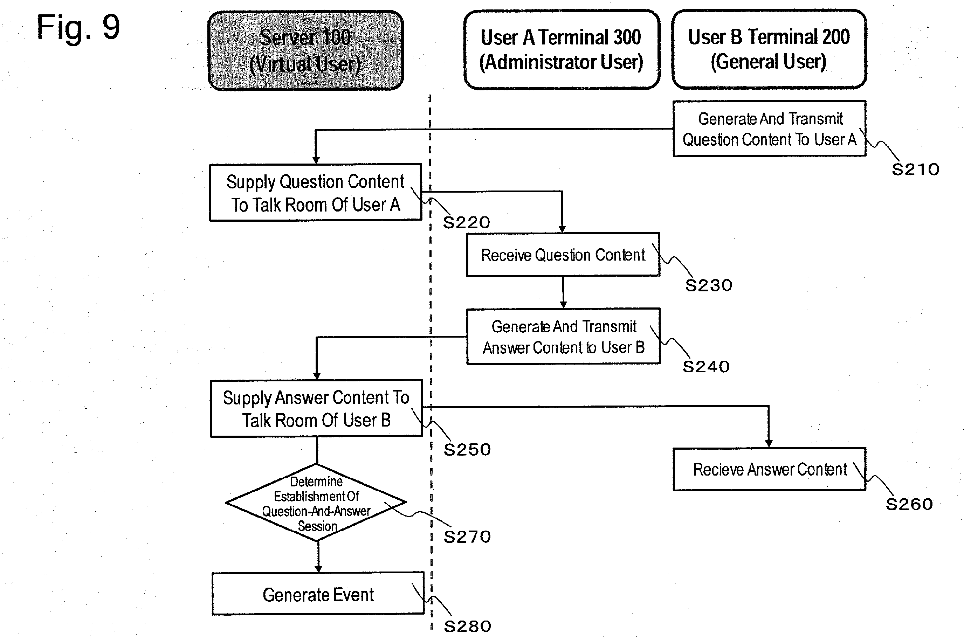

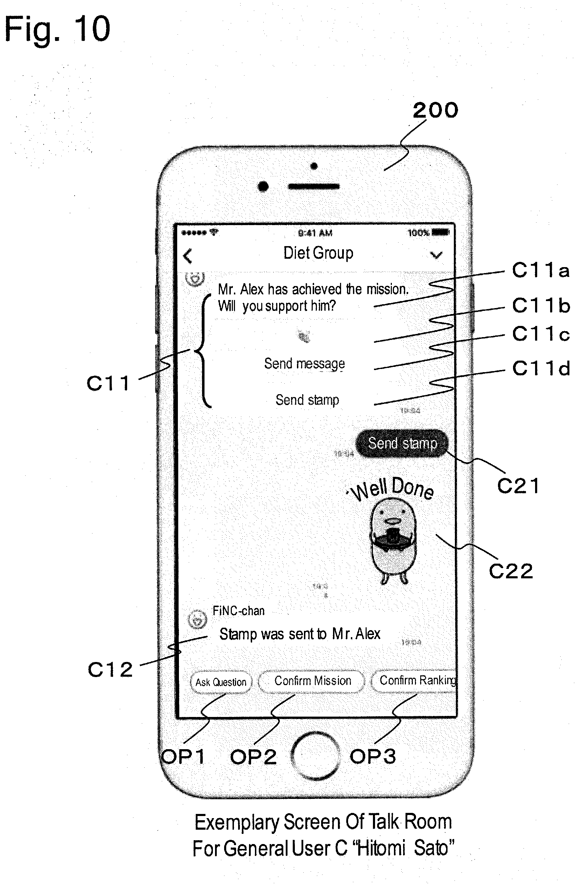

Referring now to Figs. 7 - 12, an information processing method will be described according to one embodiment. Figs. 7 - 9 are exemplary flow diagrams of steps executed by the information processing method according to one embodiment. Among these charts, Fig. 7 shows the overall processing flow, and event generation processing will be particularly described in detail in the processing flows of Figs. 8 and 9. Figs. 10 - 12 in turn show exemplary screen images implemented in the information processing method according to one embodiment and displayed on a user terminal.

### 1. Overall Processing Flow

Fig. 7 shows the overall processing flow of the information processing method according to one embodiment. As shown, the overall processing flow is executed by interactions among a server 100 (particularly, a virtual user implemented in the virtual user unit 140), a user terminal 300 of an administrator user A, a user terminal 200 of a general user B, and a user terminal 200 of a general user C.

First, an action associated with the administrator user A and general user B is executed to cause the server 100 to generate an event (S10). In response, the reception unit 138 of the server 100 receives the action. In response to the received action, the virtual user vb causes an event associated with the administrator user A and general user B to be generated in the event generation unit 141 (S20). As described later in Fig. 8 onward, the generation of the event may be triggered, for example, by the achievement of a mission by a general user, or triggered by the establishment of a question-and-answer session with an answer of the administrator user to a question from a general user. In addition, the generation of an event is determined by the determination unit 143.

After the event is generated, the virtual user vc generates prompt content for prompting the general user C to make communications with the general user B in the prompt content generation unit 145. Together, the virtual user vc supplies the generated prompt content to the talk room of the general user C in the prompt content supply unit 147 (S30). The operations at S20 and S30 are to be executed in cooperation with the virtual user vb of the general user B and the virtual user vc of the general user C.

The general user C receives the prompt content supplied from the virtual user vc in the talk room through the communication unit 210 (S40). In accordance with the received prompt content, the general user C generates reaction content destined to the general user B in the input editing unit 240, and transmits the reaction content to the server 100 through the communication unit 210 (S50). At this time, the reaction content contributed by the general user C is reflected to the talk room of the general user C.

The content supply unit 134 supplies the reaction content received from the general user C only to the talk room of the general user B (S60). Simultaneously with this, the virtual user vb of the general user B may function to censor details of the content in the virtual user unit 140. Specifically, the virtual user may parse the reaction content to check whether it contains inadequate contents. The check may be made through natural language processing or image analysis processing to confirm that the contents do not fall into a slander, they do not violate public order and morals, and so on by matching the content with information previously stored in the storage unit 190. Then, the general user B receives the reaction content supplied from the general user C in the talk room through the communication unit 210 (S70).

Subsequent to the action content supplied to the talk room of the general user B at S60, the virtual user vb generates prompt content for prompting the general user B to communicate with the general user C in the prompt content generation unit 145. Then, the virtual user vb supplies the prompt content to the talk room of the general user B in the prompt content supply unit 147 (S80). As a result, the general user B receives the prompt content supplied from the virtual user vb in the talk room through the communication unit 210 (S90). Afterward, the general user B, who has received the prompt content, can now supply response content to the talk room of the general user C in response to the prompt content through the mediation of the virtual user vc (not shown).

In this way, communications (messaging) are made between the general user B and the general user C. The messaging is always performed through the mediation of the virtual users vb, vc. Thus, the messaging is supported by the virtual users vb, vc, particularly as done at S30 and S80. For this reason, a general user can make communications without disclosing unnecessary personal information. In addition, content as deemed inadequate can be deleted through the censorship performed by the virtual user, such that the content is not supplied to the talk room. It is therefore possible to avoid troubled communities, and/or a particular user subjected to concentrated attacks. As a conclusion, a sound community can be autonomously maintained.

### II. Event Generation Processing Flow

The generation of an event by the event generation unit 141 will be described in connection with S10 and S20 of Fig. 7. The generation of an event can be triggered by (i) the achievement of a mission by a general user, or (ii) an answer of an administrator user to a question from a general user. Fig. 8 shows a processing flow for generating an event for a mission registered by the administrator user A, where the event is triggered by an event achieved by the general user B. Fig. 9 shows a processing flow for generating an event, where the event is triggered by the administrator user A answering to question content transmitted from the general user B to the administrator user A through the transmission of answer content. For simplicity, Figs. 8 and 9 shows only one general user B.

In the exemplary generation of an event in Fig. 8, the administrator user A first registers a mission associated with a community using the registration unit 250 of the user terminal (S110). A mission refers to a task forced to all or part of general users who belong to a community. A user who has been associated with a mission, is to execute an action for achieving the mission.

Examples of the mission in "Diet Group" community may be "Lose weight by 3 kilos in one month," "Walk a total of 50,000 steps or more in one week," "Do two sets of 10 squats a day," "Keep daily calorie intake not more than 1500 kcal," and the like. The administrator user A can register a variety of information related to a mission on a mission setting screen provided by the registration unit 250. For example, the administrator user A can set mission start/end date; duration (such as one month and one day), parameters (such as weight, number of steps, number of times squats should be done, and calories), value required for achievement (such as 3 kilos, 50,000 steps, twice, and 1500kcal), incentives (such as 500 points and 10 points), and the like. Other than the foregoing, video content (such as an explanation video showing how to do squats) created by the administrator user may be included as a description of what a mission is like. A mission is received by the mission registration unit 136 of the server 100, and stored as mission information (S120).

The general user B takes an associated action to the registered mission during its duration (S130). In response, the reception unit 138 of the server 100 receives the action taken by the general user B each time it is taken (S140). Information on the taken action is stored by the reception unit 138 of the server 100 as action history information. Information on the taken action is registered by the registration unit 250 of the user terminal. Specifically, the user may directly enter the information in the input editing unit 240. The information may be sensed from a sensor unit such as an acceleration sensor equipped in the user terminal 200. Alternatively, the information may be captured from a wearable device or a pre-set device which can be cooperated with the user terminal 200.

In the foregoing example, if the mission instructs to "lose weight by 3 kilos in one month," the general user B measures the weight every day, and enters the value from the input editing unit 240. Alternatively, each time the weight is measured with a pre-set weight meter possessed by the general user B, the weight may be directly communicated to the server 100 through a network. Likewise, if the mission instructs to "walk a total of 50,000 steps or more in one weak," the number of steps per day, sensed by the sensor unit 26 (particularly, the acceleration sensor) of the user terminal 200, may be periodically communicated to the server 100. If the mission instructs to "do two sets of 10 squats a day," the general user B may count the number of squats the general user B actually tries along the contents of a squat video specified by the administrator user A, and synchronize with the server 100. In this regard, the squat video may be configured to have a function which allows a general user to register the number of tries in association. If the mission instructs to "Keep daily calorie intake not more than 1500 kcal," images of daily meals may be taken by the imager unit 24 and communicated to the server 100.

In response to the received action, the virtual user vb determines the achievement of the mission in the determination unit 143 (S150). Then, if the achievement of the mission is determined, the virtual user vb generates an event in the event generation unit 141 (S160 and S20 in Fig. 7).

In the foregoing example, when the mission instructs to "lose weight by 3 kilos in one month," the mission is determined to be achieved if the general user B has lost the weight by three kilos or more in one month. Similarly, when the mission instructs to "walk a total of 50,000 steps or more in one week," the mission is determined to be achieved if the total number of steps, as measured by the user terminal 200 in one week, exceeds 50,000 steps. When the mission instructs to "do two sets of 10 squats everyday," the mission is determined to be achieved if the number of tries per day made by the general user B, associated with the squat video specified by the administrator user A, exceeds two. When the mission instructs to "keep daily calorie intake not more than 1500 kcal," the mission is determined to be achieved if the total of intake calorie, calculated by analyzing the images of meals eaten by the general user B on one day, is 1500 kcal or less.

In response to the generation of an event triggered by the achievement of the mission by the general user B, the content supply unit 134 of the server supplies the achievement of the mission by the general user B to each talk room of the administrator user A and general user B (S170). Content indicative of the achievement of the mission by the general user B is displayed on the screens of the respective talk rooms of the administrator user A and general user B (S180 and S190). Simultaneously, the virtual user vc supplies prompt content to the talk room of the other general user C in association with the content indicative of the achievement of the mission by the general user B (S30 in Fig. 7).

In the exemplary generation of an event in Fig. 9, question content from the general user B to the administrator user A is first generated by the input editing unit 240 of the user terminal of the general user B, and transmitted to the server 100 (S210). At this time, the question content is reflected to the talk room of the general user B. In the server 100, the question content is supplied to the talk room of the administrator user A by the content supply unit 134 (question content supply unit) (S220). Simultaneously, the virtual user unit 140 may function to censor whether the question content contains inadequate contents.

The administrator user A receives the question content from the general user B in his own talk room (S230). Then, in response to the received question content, the administrator user A generates answer content destined to the general user B in the input editing unit 240 of the user terminal of the administrator user A, and transmits the answer content to the server 100 (S240). At this time, the answer content is reflected to the talk room of the administrator user A. In the server 100, the answer content is supplied to the talk room of the general user B by the content supply unit 134 (answer content supply unit) (S250). Simultaneously, the virtual user unit 140 may function to sensor whether the answer content contains inadequate contents.

The general user B receives the answer content from the administrator user A in his own talk room (S260). On the other hand, on the server 100 side, the virtual user va determines in the determination unit 143 that question/answer exchanges (i.e., question-and-answer session) is established between the general user B and the administrator A (S270). Then, upon determining the establishment of the question-and-answer session, the virtual user va generates an event in the event generation unit 141 (S280). Simultaneously, the virtual user also supplies the prompt content to the talk room of the other general user C in association with the question content and the answer content (S30 in Fig. 7).

### III. Exemplary Talk Room Screens

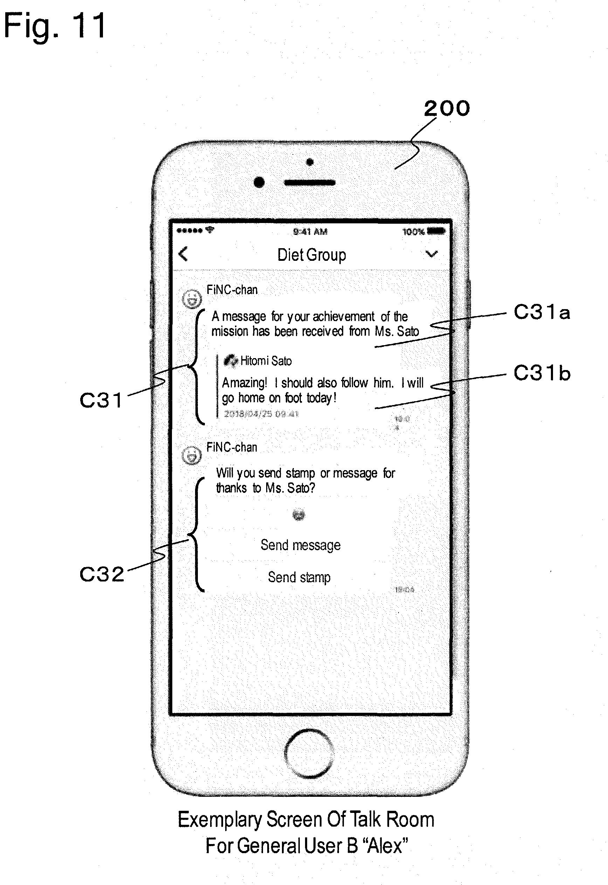

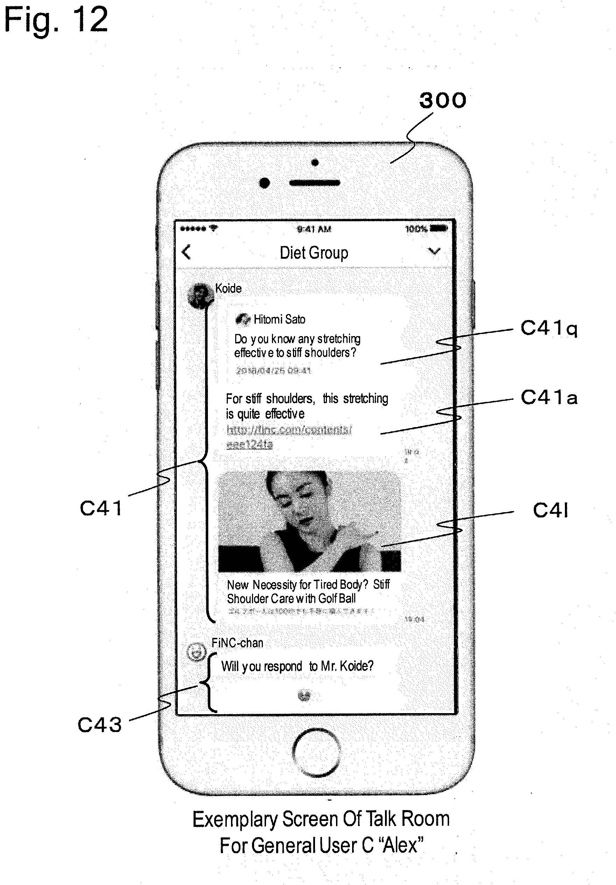

Referring to Figs. 10 - 12, a description will be made for exemplary screens of the talk room displayed on the user terminal, as implemented by the information processing method according to one embodiment. Figs. 10 and 11 show exemplary messaging in talk rooms displayed on the respective user terminals of the general user B and general user C in the "Diet Group" community, in association with the overall processing flow shown in Fig. 7 and the event generation processing flow shown in Fig. 8. Fig. 12 shows exemplary messaging in the talk room also displayed on the user terminal of the general user C in the "Diet Group" community, in association with the overall processing flow shown in Fig. 7 and the event generation processing flow shown in Fig. 9.

More specifically, Fig. 10 shows an exemplary talk room for "Hitomi Sato" who is the general user C, while Fig. 11 shows an exemplary talk room for "Alex" who is the general user B. Also, Fig. 12 shows an exemplary talk room for "Alex" who is the general user C (in this example, the general user B is "Hitomi Sato"). Common to Figs. 10 - 12, the administrator user A is "Koide." As shown in Figs. 10 - 12, on the screen of the talk room of the user terminal 200 of a general user, content supplied by a virtual user "FiNC-chan" or the administrator user A "Koide" is displayed on the left column in a time-series order. Also, content supplied by the general user A or B himself is displayed on the right column in a time series order. In this regard, the manner of displaying the talk room is not limited to the above examples. Content supplied by the virtual user "FiNC-chan" may be displayed on the right column in the talk room.

In the exemplary screens of the talk rooms in Figs. 10 and 11, a mission has been previously registered by the administrator user A "Koide" (S110, S120 in Fig. 8). As a result of an action taken by the general user B "Alex" for the mission (S130, S140 in Fig. 8, S10 in Fig. 7), the mission is achieved (S150 in Fig. 8), triggering an event to occur (S160 in Fig. 8, S20 in Fig. 7).

In the exemplary screen of Fig. 10, the virtual user "FiNC-chan" generates prompt content C11a for the general user C "Hitomi Sato," saying "Mr. Alex has achieved the mission. Will you support him?" which is supplied to the talk room (S30, S40 in Fig. 7). Together with this, options for a reaction are also displayed in the talk room as part of prompt content C11, including a clapping-hand icon C11b indicative of "good," "Send Message" C11c, and "Send Stamp" C11d.

The general user C "Hitomi Sato" has selected "Send Stamp" C21 from among the options C11b - C11d for the reaction provided from the virtual user. Then, she has selected a "Well Done" stamp C22, as a reaction content, which includes "Well Done" displayed together with an image, and transmitted the same (S50 in Fig. 7). In response, the virtual user "FiNC-chan" has supplied the stamp C22 to the talk room of the general user B "Alex" as well (S60, S70 in Fig. 7). As a result, the talk room for the general user C "Sato Hitomi" is supplied with content C12 generated by the virtual user "FiNC-chan" saying "Stamp was sent to Mr. Alex," which is displayed in the screen.

Subsequently, when the general user C "Hitomi Sato" is to take a further action in the talk room of the "Diet Group," she will select an option from "Ask Question" OP1, "Confirm Mission" OP2, "Confirm Ranking" OP3, and the like displayed on the bottom of the screen. For example, if she selects "Ask Question" OP1, she can ask a question to the administrator user A and receive a specific answer (advice) from the administrator user A. When she selects "Confirm Mission" OP2, she can confirm specific contents of a mission associated with the general user C "Hitomi Sato," registered by the administrator user A. When she select "Confirm Ranking" OP3, she can confirm the degree of achievement for the mission in a ranking form within the "Diet Group" community.

Fig. 10 shows an exemplary screen of the talk room for the general user C "Sato Hitomi," whereas Fig. 11 shows an exemplary screen of the talk room for the general user B "Alex." As described above, in the exemplary screen of Fig. 10, the general user C "Hitomi Sato" has transmitted the stamp C22 as a reaction content for the achievement of the mission by the general user B "Alex." On the other hand, in the exemplary screen of Fig. 11, the general user C Hiromi Sato" has transmitted a text-based message C31b as a reaction content.

In the exemplary screen of Fig. 11, the virtual user "FiNC-chan" notifies that a message destined to the general user B "Alex" has been received from the general user C "Hitomi Sato" (S60 in Fig. 7), and the content C31 is displayed on the screen (S70 in Fig. 7). The content C31 includes a message C31a of the virtual user "FiNC-chan" saying "A message for your achievement of the mission has been received from Ms. Sato," and reaction content C31b of the general user C "Hitomi Sato" saying "Amazing! I should also follow him. I will go home on foot today!." In this way, in the exemplary screen of Fig. 11, the reaction content by the general user C Hitomi Sato" is displayed on the left column of the screen in association with the messaging by the virtual user "FiNC-chan." Thus, even if a message is from the general user C "Hitomi Sato," it is mediated by the virtual user "FiNC-chan." In this way, communications can be readily made by the virtual user which serves to mediate the communications, even if the general users B, C are not actually acquaintances. Consequently, the community is activated.

Next, the virtual user "FiNC-chan" generates prompt content (message) saying "Will you send an appreciative stamp or message to Ms. Sato?," and supplies the message to the talk room (S80 in Fig 7). As is the case with C11, options for a reaction, i.e., a clapping-hand icon indicative of "good," "Send Message", and "Send Stamp" are supplied to the talk room together with the message. As a result, the prompt content C32 is displayed in the talk room of the "Diet Group" for the general user B "Alex" (S90 in Fig. 7). The general user B "Alex" responds to the prompt content C32 from the virtual user "FiNC-chan," causing a further communication to be made with the general user C "Hitomi Sato" through the virtual user to activate the community.

The exemplary screens of the talk rooms in Figs. 10 and 11 described above relate to the event generation processing flow involved in the achievement of a mission shown in Fig. 8, whereas the exemplary screen of the talk room in Fig. 12 relates to the event generation processing shown in Fig. 9.

With respect to the exemplary screen of Fig. 12, the general user B "Hitomi Sato" generates question content C41q to the administrator user A "Koide," saying "Do you know any stretching effective to stiff shoulders?" which is transmitted to the server (S210 in Fig. 9). The administrator user A "Koide," who has received the question content C41q through the server, generates answer content C41a saying "For stiff shoulders, this stretching is quite effective" which is transmitted to the server (S240 in Fig. 9). The answer content C41a also includes link information C411 for a web page entitled "New Necessity for Tired Body? Stiff Shoulder Care with Golf Ball" and a video. As a result of these, a question-and-answer session has been established between the general user C "Hitomi Sato" and the administrator user A "Koide" (S270 in Fig. 7), causing an event to be generated (S290 in Fig. 9 and S20 in Fig. 7).

Like the exemplary screen of Fig. 12, the talk room screen of the general user C "Alex" displays content C41 involved in a sequence of the question-and-answer session, including the question content C41q and the answer content c41a (also including the link information C41l). Specifically, the messaging by the general user C "Hitomi Sato" is displayed on the left column of the talk room in association with the messaging by the administrator user A "Koide." In this way, the general user C "Alex" can confirm the question-and-answer session between the general user B "Hitomi Sato" and the administrator user A "Koide."

Next, the virtual user "FiNC-chan" generates prompt content C43 saying "Will you respond to Mr. Koide?" which is supplied to the talk room for the general user B "Alex" (S30, S40 in Fig. 7). Also, options for a reaction, i.e., a clapping-hand icon indicative of "good," "Send Message," and "Send Stamp" are displayed in the talk room as part of the prompt content C43, as is the case with C11, C31 (S40 in Fig. 7).

With the embodiment of the present disclosure, communities can be activated through the autonomous mediation of a virtual user in a community service within a virtual space. Particularly, by supporting the maintenance and improvement of the relationship between an administrator user and general users, and between general users, the number of members belonging to a community can be effectively increased, accompanied with improvement in active user ratio. Also, by providing the administrator user with an effective operation of community services through the mediation of the virtual user, the administrator user can be released from burdens involved in the maintenance and administration of the community. Further, general users can be encouraged to utilize community services without hesitation through the mediation of the virtual user, obviating such situations as a troubled community, and concentrated attacking to a particular general user. In addition, unnecessary private data of general users can be prevented from being disclosed.

The foregoing embodiment is a mere illustration for facilitating the understanding of the present invention, and is not provided to interpret the present invention in a limiting sense. It should be understood that the present invention can be modified and improved without departing from the spirit and scope thereof, and that the present invention also includes its equivalents.

## Claims

1. An information processing method, wherein:
a community is associated with users including an administrator user and a plurality of general users, and with a virtual user for supporting the users, said method comprising the steps, executed by a computer, of:
generating an event associated with the administrator user and a first general user; and
in response to the generated event, causing the virtual user to generate prompt content for prompting a second general user to communicate with the first general user, and supply the prompt content to the second general user in the community.

2. A method according to claim 1, further comprising the step of:
in response to the supplied prompt content, supplying reaction content created by the second general user to the first general user in the community.

3. A method according to claim 1 or 2, further comprising the steps of:
accepting a registration of a mission by the administrator user, the mission being associated with the community;
receiving an action taken on the mission by the first general user; and
in response to the received action, determining the achievement of the mission;
wherein the event is generated in accordance with the achievement of the mission.

4. A method according to any of claims 1 through 3, further comprising the steps of:
supplying question content created by the first general content to the administrator user in the community; and
supplying answer content created by the administrator user in response to the question content supplied to the first general user in the community,
wherein the event is generated in response to the establishment of a question-and-answer session made up of the question content and the answer content.

5. A method according to any of claims 1 through 4, wherein said community is implemented as a talk room by an instant messaging application, and
on a screen of the talk room displayed on a terminal of the second general user, messaging by the second general user is displayed on a first column, and messaging by the administrator user and the virtual user is displayed on a second column in a time-series order.

6. A method according to claim 5, wherein:
on the screen of the talk room displayed on the terminal of the second general user, messaging by the first general user is also displayed on the second column in association with messaging by the virtual user and the administrator user.

7. An information processing device, comprising:
a management unit for associating users including an administrator user and a plurality of general user with a virtual user for supporting the users in a community;
an event generation unit for generating an event associated with the administrator user and a first general user; and
a prompt content supply unit for causing the virtual user to, in response to the generated event, generate prompt content for prompting a second user to communicate with the first general user, and supply the prompt content to the second general user in the community.

8. An information processing device according to claim 7, further comprising:
a reaction content supply unit for supplying reaction content, created by the second general user in response to the supplied prompt content, to the first user in the community.

9. An information processing device according to claim 7 or 8, further comprising:
a mission registration unit for accepting a registration of a mission by the administrator user, said mission being associated with the community;
a reception unit for receiving an action taken on the mission by the first general user; and
a determination unit for determining the achievement of the mission in response to the received action,
wherein the event is generated in the event generation unit in accordance with the achievement of the mission.

10. An information processing device according to any of claims 7 through 9, further comprising:
a question content supply unit for supplying question content created by the first general user to the administrator user in the community; and
an answer content supply unit for supplying answer content created by the administrator user in response to the supplied question content, to the first general user in the community,
wherein the event is generated in the event generation unit in response to the establishment of a question-and-answer session made up of the question content supply unit and the answer content supply unit.
